**Europäisches Patentamt**

**European Patent Office**

**Office européen des brevets**

(11) Veröffentlichungsnummer: **0 218 122**
**A1**

(12) ## EUROPÄISCHE PATENTANMELDUNG

(21) Anmeldenummer: 86112722.3

(51) Int. Cl.⁴: **A61F 5/00** , A47K 13/24

(22) Anmeldetag: 15.09.86

(30) Priorität: 26.09.85 CH 4186/85

(43) Veröffentlichungstag der Anmeldung:
15.04.87 Patentblatt 87/16

(84) Benannte Vertragsstaaten:
AT BE DE FR GB LU NL SE

(71) Anmelder: Fobe AG
Durnacheli
CH-6074 Giswil(CH)

(72) Erfinder: Schaudig, Helmut, Prof. Dr. med.
Durnacheli
CH-6074 Giswil(CH)

(74) Vertreter: Blum, Rudolf Emil Ernst et al
c/o E. Blum & Co Patentanwälte Vorderberg 11
CH-8044 Zürich(CH)

(54) Einrichtung zum Stützen der Beckenbodenmuskulatur.

(57) Die Einrichtung besteht im wesentlichen aus einem ersten Organ (5), das an einem zweiten Organ (6) vorgesehen ist. Das zweite Organ (6) kann dabei mit dem ersten Organ (5) einstückig ausgebildet oder fest bzw. beweglich mit diesem verbunden sein.

Das erste Organ (5) hat einen konvexen Abschnitt, auf den der Beckenboden auflegbar ist, um eine Auswölbung des Beckenbodens zu vermeiden und die Stuhlentleerung zu erleichtern.

Fig. 2

EP 0 218 122 A1

## Einrichtung zum Stützen der Beckenbodenmuskulatur

Die vorliegende Erfindung betrifft eine Einrichtung zum Stützten der Beckenbodenmuskulatur bei der natürlichen Stuhlentleerung.

Durch Erschlaffung bzw. Ueberdehnung der Beckenbodenmuskulatur wird die natürliche Lage des Enddarmes und damit die natürliche Stuhlentleerung bei vielen Menschen erschwert.

Ziel der Erfindung ist es, eine Einrichtung zu - schaffen, mit der die Stuhlentleerung erleichtert wird.

Dieses Ziel wird erfindungsgemäss durch die Merkmale im kennzeichnenden Teil des Patentanspruches I erreicht.

Der mit der Erfindung erreichbare Vorteil ist im wesentlichen darin zu sehen, dass die Stützung der Beckenbodenmuskulatur durch ein Widerlager erfolgt, das im Idealfall vom Verwender allein durch entsprechendes Andrücken, die von der Steissbeinspitze zum After ziehende Muskulatur stützt.

Bei einer bevorzugten Ausführungsform ist das zweite Organ als Toilettensitz ausgebildet. Dies hat den Vorteil, dass der Verwender allein durch kräftigeres oder lockeres Sitzen, die Beckenbodenmuskulatur entsprechend stützen kann.

Bei einer anderen Ausführungsform kann das erste Organ mit einer Haltevorrichtung versehen sein, so dass das Organ an einen vorhandenen Toilettensitz befestigt werden kann.

Bei einer weiteren Ausführungsform kann das zweite Organ als auf einem Toilettensitz auflegbarer Teil ausgebildet und mit Mitteln versehen sein, so dass der Teil auf einen vorhandenen Toilettensitz aufgelegt werden kann.

Im folgenden wird die Erfindung anhand der beiliegenden Zeichnungen näher erläutert.

Es zeigen:

Fig. I eine schematische Darstellung einer Ausführungsform der erfindungsgemässen Einrichtung in Gebrauchslage;

Fig. 2 eine Draufsicht auf die in Fig. I dargestellte Ausführungsform; und

Fig. 3 bis 5 Schnitte durch andere Ausführungsformen der erfindungsgemässen Einrichtung.

Wie aus den Figuren I und 2 ersichtlich ist, handelt es sich bei der dargestellten Ausführungsform um einen Toilettensitz. Die Fig. I zeigt ferner einen Teil des menschlichen Beckens mit dem Steissbein I, dem Beckenboden 2 und dem After 3. Der Toilettensitz enthält einen ersten Abschnitt 5, der mit dem eigentlichen Sitzteil 6 einstückig ausgebildet ist. Der erste Abschnitt 5 weist einen konvexen Abschnitt 7 auf. In der Gebrauchslage liegt der Beckenboden 2 auf diesen ersten Abschnitt 5 auf. Durch diese Auflage wird verhindert, dass bei erschlaffter bzw. überdehnter Beckenbodenmuskulatur der Beckenboden 2 nach aussen gewölbt und die natürliche Stuhlentleerung erschwert wird.

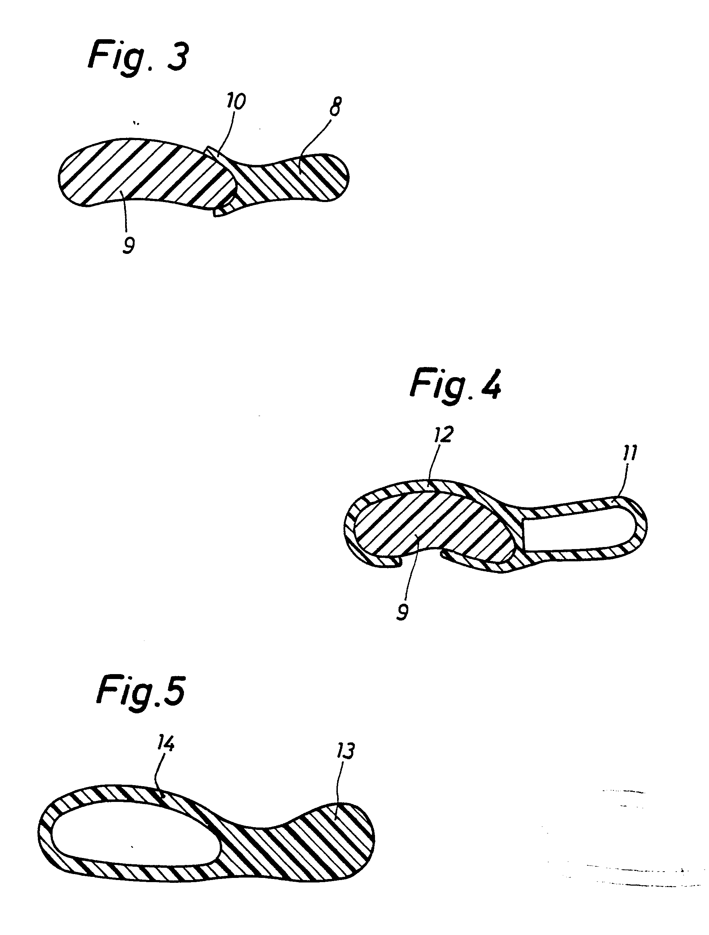

Die Fig. 3 zeigt eine andere Ausführungsform der erfindungsgemässen Einrichtung, bei der zur Stützung des Beckenbodens ein erstes Organ 8 vorgesehen ist, das an einem zweiten Organ 9 befestigt ist. Das erste Organ 8 unterscheidet sich von dem in Fig. I und 2 dargestellten Abschnitt 5 lediglich dadurch, dass es eine Haltevorrichtung, bestehend aus einem Halteabschnitt I0 und einem Be festigungsmittel oder einem Scharnier (nicht dargestellt) aufweist. Wie aus dieser Fig. 3 ersichtlich ist, kann das zweite Organ 9 ein Toilettensitz oder ein mindestens eine Stütze (nicht dargestellt) aufweisendes Organ sein, das insbesondere bei Stehbecken anwendbar wäre. Das erste Organ 8 kann ein Voll-oder Hohlkörper sein. Bei der Ausbildung als Hohlkörper kann das erste Organ 8 mit einem Vibrator oder einer Vorrichtung versehen werden, um eine Vibration oder elektrische Stimulation der Beckenbodenmuskulatur zu bewirken.

Die in Fig. 4 dargestellte Ausführungsform der erfindungsgemässen Einrichtung besteht aus einem ersten Organ II mit einem Halteabschnitt I2, die einstückig miteinander ausgebildet sind. In Fig. 4 ist das erste Organ II als Hohlkörper dargestellt. Selbstverständlich ist es möglich, dieses erste Organ II als Vollkörper auszubilden.

Das zweite Organ I2 ist so ausgestaltet, dass es mindestens auf den Hinterteil eines vorhandenen Toilettensitzes aufsetzbar ist. Das zweite Organ I2 kann auch so ausgestaltet werden, dass es über einen vorhandenen Toilettensitz gezogen werden kann. Durch die vorstehend beschriebene Ausführung kann die in Fig. 4 dargestellte Einrichtung zusammengelegt werden und eignet sich in vorteilhafter Weise für Reisen.

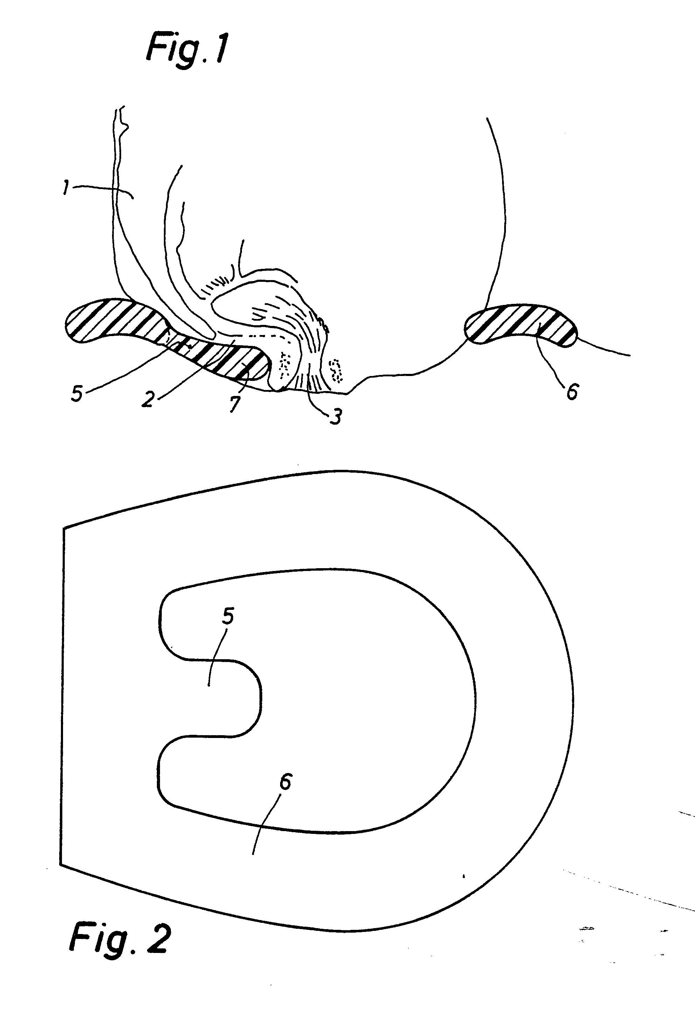

Bei der in Fig. 5 dargestellten Ausführungsform ist das erste Organ I3 ein Vollkörper, der mit dem als Hohlkörper ausgebildeten zweiten Organ I4 einstückig ausgebildet ist. Diese Ausführungsform bringt vor allem eine Gewichtseinsparung gegenüber der in Fig. 2 dargestellten Einrichtung. Ferner kann bei dieser Ausführungsform ein Vibrator oder eine Vorrichtung (nicht dargestellt) vorgesehen werden, um eine Vibration oder elektrische Stimulation der Beckenbodenmuskulatur zu bewirken.

## Ansprüche

1. Einrichtung zum Stützen der Beckenbodenmuskulatur bei der natürlichen Stuhlentleerung, gekennzeichnet durch ein Organ (5,8,11,13), das einen an den Beckenboden (2) anlegbaren konvexen Abschnitt (7) aufweist.

2. Einrichtung nach Anspruch 1, gekennzeichnet durch einen Vibrator, um eine Vibration der Muskulatur zu bewirken.

3. Einrichtung nach Anspruch 1, gekennzeichnet durch eine Vorrichtung, um eine elektrische Stimulation der Muskulatur zu bewirken.

4. Einrichtung nach Anspruch 1, gekennzeichnet durch ein zweites Organ (6,9,14), an dem das erste Organ (5,8,11,13) vorgesehen ist.

5. Einrichtung nach Anspruch 4, dadurch gekennzeichnet, dass das erste oder zweite Organ ein Vollkörper ist.

6. Einrichtung nach Anspruch 4, dadurch gekennzeichnet, dass das erste oder zweite Organ ein Hohlkörper ist.

7. Einrichtung nach Anspruch 4, dadurch gekennzeichnet, dass das erste und zweite Organ ein Hohlkörper ist.

8. Einrichtung nach Anspruch 4, dadurch gekennzeichnet, dass das erste und zweite Organ ein Vollkörper ist.

9. Einrichtung nach Anspruch 6 oder 7, dadurch gekennzeichnet, dass der Hohlkörper elastisch verformbar ist.

10. Einrichtung nach Anspruch 9, gekennzeichnet durch mindestens einen Anschlussteil, um den Hohlkörper mit einem gasförmigen oder hydraulischen Medium zu füllen.

11. Einrichtung nach Anspruch 4, dadurch gekennzeichnet, dass das erste und zweite Organ - (5,6; 13,14) ein stückig ausgebildet sind.

12. Einrichtung nach Anspruch 4, dadurch gekennzeichnet, dass das erste und zweite Organ - (8,9,11) fest oder beweglich miteinander verbunden sind.

13. Einrichtung nach Anspruch 11, gekennzeichnet durch Mittel (10,12) zum Verbinden der Organe.

14. Einrichtung nach Anspruch 13, dadurch gekennzeichnet, dass die Mittel ein Scharnier umfassen, um das erste Organ (8) in und aus eine Gebrauchslage zu schwenken.

15. Einrichtung nach Anspruch 13, dadurch gekennzeichnet, dass die Mittel mindestens ein elastisches Element umfassen, um das erste Organ elastisch am zweiten Organ zu halten.

16. Einrichtung nach einem der Ansprüche 4-12, dadurch gekennzeichnet, dass das zweite Organ - (6) ein Toilettensitz ist.

17. Einrichtung nach einem der Ansprüäche 4-12, dadurch gekennzeichnet, dass das zweite Organ als ein mindestens eine Stütze aufweisender Sitz ausgebildet ist.

18. Einrichtung nach Anspruch 1, gekennzeichnet durch eine Haltevorrichtung, um das Organ - (8,11) an einem Toilettensitz zu befestigen.

19. Einrichtung nach Anspruch 18, dadurch gekennzeichnet, dass das Organ (8,11) und die Haltevorrichtung einstückig ausgebildet sind.

20. Einrichtung nach Anspruch 18, dadurch gekennzeichnet, dass die Haltevorrichtung eine Klemmvorrichtung aufweist, um das Organ an einem Toilettensitz festzuklemmen.

21. Einrichtung nach Anspruch 4, dadurch gekennzeichnet, dass das zweite Organ (12) als auf einen Toilettensitz auflegbarer Teil ausgebildet und mit Mitteln versehen ist, um den Teil auf den Toilettensitz zu halten.

Fig.1

Fig. 2

*Fig. 3*

10

8

9

*Fig. 4*

12

11

9

*Fig. 5*

14

13

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-2 985 171 (WIEDERMAN) <br><br> * Insgesamt * | 1,4-6, 12,13, 15-21 | A 61 F 5/00 <br> A 47 K 13/24 |
| Y | | 2 | |
| | --- | | |
| Y | DE-A-2 729 987 (WOLF) <br> * Anspruch 1 * | 2 | |
| | --- | | |
| A | US-A-2 534 664 (GOTTLIEB) | | |
| | --- | | |
| A | US-A-2 256 994 (WARSHAW) | | |
| | ----- | | |

| RECHERCHIERTE SACHGEBIETE (Int. Cl.4) |
|---|
| A 61 F <br> A 61 G <br> A 61 H <br> A 47 K |

Der vorliegende Recherchenbericht wurde für alle Patentansprüche erstellt.

| Recherchenort | Abschlußdatum der Recherche | Prüfer |
|---|---|---|
| DEN HAAG | 07-01-1987 | STEENBAKKER J. |